# EUROPEAN PATENT APPLICATION

(11) **EP 4 583 119 A1**
(43) Date of publication of application: **09.07.2025**
(21) Application number: 24150672.4
(22) Date of filing: 08.01.2024
(51) Int. Cl.: G16H 40/63, G16H 30/20, G16H 30/40, G16H 40/67, G16H 50/20, A61B 5/00, G06N 3/00, G06N 5/00, G06N 7/00, G16H 50/70

(54) **MODULAR CONTEXT SUITE**

(71) Applicant: Koninklijke Philips N.V., 5656 AG Eindhoven (NL)
(72) Inventor: JOCKEL, Sascha Andreas, Eindhoven (NL); HAWELLEK, Benjamin, Eindhoven (NL); WIEBERNEIT, Nataly, 5656AG Eindhoven (NL)
(74) Representative: Philips Intellectual Property & Standards

(57) **Abstract**

A selection of one of a plurality of potential contextual display applications is received for the display of a processed image at a local device. An image from an imaging device is received at a server, and the image at the server is processed by applying an image processing routine based on the selected one of the plurality of potential contextual display applications. Typically, each potential contextual display application has an associated distinct processing routine. The processed image is then transmitted to the local device for display and displayed at the local device in the context of the selected contextual display application.

## Description

### FIELD

The present invention generally relates to a context suite for displaying clinical results. In particular, the present invention relates to a modular and dynamically extendable application to consolidate and display results from various independent clinical applications relating to a contextually similar clinical problem.

### BACKGROUND

In clinical decision making, medical staff usually takes numerous inputs into account. Software support, including AI algorithms, have the potential to support this process, but a holistic approach is also required - isolated, individual algorithms have limited value. Since currently available clinical applications usually only answer partial aspects of a clinical question, numerous clinical applications are required to assess the (health) condition of the patient holistically. This in turn is inconvenient and potentially leads to slow adoption of AI and other software-based support platforms.

Accordingly, research has focused on how to provide medical staff with AI functionality at a point of acquisition with a focus on mobile X-ray imaging.

It has been observed that in many hospitals, physicians interpret x-ray images themselves due to the time pressure associated with current clinical questions. The radiology department may be responsible for full reporting, but that is usually done at a later stage. Image interpretation in radiology thereby becomes a bottleneck, and radiologists, as specialist readers, are only consulted by the physicians directly for difficult cases and decisions.

Clinical decision-making needs a holistic approach, since individual algorithms provide limited assistance in clinical and operational environments. Since algorithms usually answer only sub aspects of a defined clinical question, numerous clinical applications that answer mostly only sub-questions are required to assess the patient's (health) condition holistically. Accordingly, expertise in use of a wide variety of relevant algorithms is required in addition to medical expertise in order to properly leverage existing tools.

Currently, the market is full of AI algorithms offered by different vendors, but typically each algorithm is offered in a separate tool and only answers a very specific clinical sub-question. In order to be able to make a holistic decision, several tools would have to be used, all of which would have to be embedded individually in the clinical workflow. This integration of a wide variety of tools has a negative overall effect on the customer's workflows and increases the complexity for the user, who is already under time pressure.

Organizational and individual users of such AI algorithms would like to achieve a) faster image interpretation and more confident clinical decisions, b) support for a common and consistent level of interpretation quality across departments and c) training of residents and new staff. However, practically, potential requirements for a solution from the user's point of view would be a) to display only relevant information, b) to enable switching on and off of supplementary information and overlays, c) that the tool should be close to the modality and available in the office and, d) multiple relevant clinical applications, including relevant AI software tools, for the given clinical context should be available in one tool.

### SUMMARY

This invention proposes a software platform referred to herein as a context suite. The context suite described herein is a dynamically extendable web application to support physicians and technologists, such as intensive care physicians and radiologists, with additional information derived from clinical image data in order to answer clinical questions and assess patient health status in a given context. Such assessments are then consolidated into a single tool for use at a point of care.

Such a platform combines multiple clinical applications in a single tool, providing medical staff with flexible access to relevant (and often AI-based) image analysis results at the point of acquisition or in physicians' offices. The platform described may allow flexible onboarding of new algorithms, making it easy to stay up to date with the latest advances in decision support algorithms, while shortening cycle time for solution updates and new product launches.

A context suite then provides the user with one platform that compiles multiple clinical applications in a single tool. Such a context suite provides the medical staff flexible access to relevant functionality, be it directly at the point of acquisition or in the physicians' office, wherever it is needed.

Due to the easy expandability and the simple onboarding of additional applications, users, such as hospitals, can more easily stay up to date with the latest advances in decision support algorithms. This is also ensured by a decoupling from the X-ray system releases and allows for shortened cycle time for solution updates and new product launches. For example, where the context suite is driven by cloud based software, only the software provided in the cloud must be updated or released and the new applications may then be loaded directly into the context suite based on an updated customer's subscription. Should new requirements arise in the future, such as a pandemic that requires quick actions, such a platform can react to the new clinical requirements much more quickly.

In some embodiments, a method is provided for contextually processing images. The method includes receiving a selection of one of a plurality of potential contextual display applications for the display of a processed image at a local device. The method then includes receiving an image from an imaging device at a server, and processing the image at the server by applying an image processing routine based on the selected one of the plurality of potential contextual display applications. Typically, each potential contextual display application has an associated distinct processing routine.

The method then proceeds with transmitting the processed image to the local device for display, and displaying the processed image at the local device in the context of the selected contextual display application.

In some embodiments, the receiving of the image at the server is from a DICOM store, wherein the DICOM store receives the image from the imaging device, the imaging device being a networked imaging device.

In some embodiments, the plurality of potential contextual display applications operate within a context suite, and wherein the potential contextual display applications are installed interchangeably within the context suite.

In some such embodiments, upon installation of the context suite at the local device, the instance of the context suite is paired with the imaging device, such that the source of the image received at the server depends on the specific local device at which the contextual display application is selected.

In some embodiments in which the potential contextual display applications are installed interchangeably within the context suite, the plurality of potential contextual display applications are installed locally within the context suite, and a user selects one of the potential contextual display applications based on a current medical context.

In some embodiments, a first potential contextual display application of the plurality is a tube and line enhancement module and a second potential contextual display application of the plurality is a device placement assessment module, and the modules are accessible interchangeably within the context suite.

In some such embodiments, the processing routine associated with the tube and line enhancement module adjusts contrast of the corresponding processed image in order to enhance clarity of catheters, tubes, or cannulas, and the processing routine associated with the device placement assessment module detects the presence of a tube in the corresponding image and evaluates a position of the tube.

In some such embodiments, the processing routine associated with the device placement assessment module further includes segmenting the identified tube and a target region for the tube.

In some embodiments in which the potential contextual display applications are installed interchangeably within the context suite, the plurality of potential contextual display applications are available remotely and can be selected for incorporation into the context suite, and the incorporation of a contextual display application allows for use of the contextual display application within a previously installed instance of the context suite.

In some embodiments, the local device is associated with one of a plurality of contexts including an intensive care unit (ICU), emergency room (ER), a trauma center, a general radiation center, a pediatric center, a military hospital, an orthopedic center, a chest room, a screening center, a geriatric center, or an occupational therapy center, and wherein a plurality of potential contextual display applications are selected for incorporation into the context suite based on the specific context with which the local device is associated.

In some embodiments, the plurality of potential contextual display applications include a tube and line enhancement module, a device placement assessment, and a lung opacity assessment, and the image processing routine applied at the server to process the image differs based on the selected contextual display application of the plurality of potential contextual display applications.

In some such embodiments, the processing routine associated with the tube and line enhancement module adjusts contrast of the corresponding processed image in order to enhance clarity of catheters, tubes, or cannulas, wherein the processing routine associated with the device placement assessment module detects the presence of a tube in the corresponding image and evaluates a position of the tube, and wherein the processing routine associated with the lung opacity assessment generates an overlay for illustrating a lung opacity score for different locations within the processed image.

In some embodiments, an updated image processing routine can be installed at the server and implemented at the local device without any required installation at the local device.

In some such embodiments, the updated image processing routine is for identifying a presence of an infectious disease.

In some embodiments, the imaging device is a CT imaging device and the image is a three-dimensional image.

In some embodiments, imaging device is a conventional X-ray device.

In some embodiments, the method further includes presenting the image at the local device prior to receiving the selection of one of the plurality of potential contextual display applications.

In some embodiments, each distinct processing routine is an AI processing routine trained on images associated with a context related to the corresponding potential contextual display application.

Also provided is a system for contextually processing images. The system includes a memory for storing a plurality of instructions, an imaging device, a server for receiving an image from the imaging device and applying an image processing routine, and a local device for displaying a processed image received from the server.

The system also includes at least one processor that couples with the memory and is configured to execute the instructions to receive a selection of one of a plurality of potential contextual display applications for the display of a processed image at the local device, receive the image from the imaging device at the server, process the image at the server by applying an image processing routine based on the selected one of the plurality of potential contextual display applications, wherein each potential contextual display application has an associated distinct processing routine, transmit the processed image to the local device for display, and display the processed image at the local device in the context of the selected contextual display application.

In some such systems, the processor is further configured to implement a troubleshooting interface upon receiving a selection of the same at the local device.

### BRIEF DESCRIPTION OF THE DRAWINGS

FIG. 1 is a schematic illustration of a context suite according to one embodiment of the invention.
FIG. 2 is an illustration of the provision of applications to users in the context of the context suite of FIG. 1.
FIG. 3 illustrates the use of the context suite of FIG. 1 in context.
FIG. 4 is a flow chart showing a method for contextually processing images according to one embodiment of the invention.
FIG. 5 shows the receipt of an image from an imaging device in the context of the context suite of FIG. 1.
FIG. 6 shows the application of a contextually selected processing routine in the context of the context suite of FIG. 1.
FIG. 7 shows the application of a second contextually selected processing routine in the context of the context suite of FIG. 1.
FIG. 8 shows the application of a third contextually selected processing routine in the context of the context suite of FIG. 1.
FIG. 9 shows the application of the second contextually selected processing routine in the context of a standard user interface accessible by physicians.
FIG. 10 shows a troubleshooting application incorporated into the context suite of FIG. 1.
FIGs. 11-13 illustrate potential contexts to which the context suite of FIG. 1 can be applied.

### DETAILED DESCRIPTION OF THE PREFERRED EMBODIMENTS

The description of illustrative embodiments according to principles of the present disclosure is intended to be read in connection with the accompanying drawings, which are to be considered part of the entire written description. In the description of embodiments of the disclosure disclosed herein, any reference to direction or orientation is merely intended for convenience of description and is not intended in any way to limit the scope of the present disclosure. Relative terms such as "lower," "upper," "horizontal," "vertical," "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise. Moreover, the features and benefits of the disclosure are illustrated by reference to the exemplified embodiments. Accordingly, the disclosure expressly should not be limited to such exemplary embodiments illustrating some possible non-limiting combination of features that may exist alone or in other combinations of features; the scope of the disclosure being defined by the claims appended hereto.

This disclosure describes the best mode or modes of practicing the invention as presently contemplated. This description is not intended to be understood in a limiting sense, but provides an example of the disclosure presented solely for illustrative purposes by reference to the accompanying drawings to advise one of ordinary skill in the art of the advantages and construction of the invention. In the various views of the drawings, like reference characters designate like or similar parts.

It is important to note that the embodiments disclosed are only examples of the many advantageous uses of the innovative teachings herein. In general, statements made in the specification of the present application do not necessarily limit any of the various claimed disclosures. Moreover, some statements may apply to some inventive features but not to others. In general, unless otherwise indicated, singular elements may be in plural and vice versa with no loss of generality.

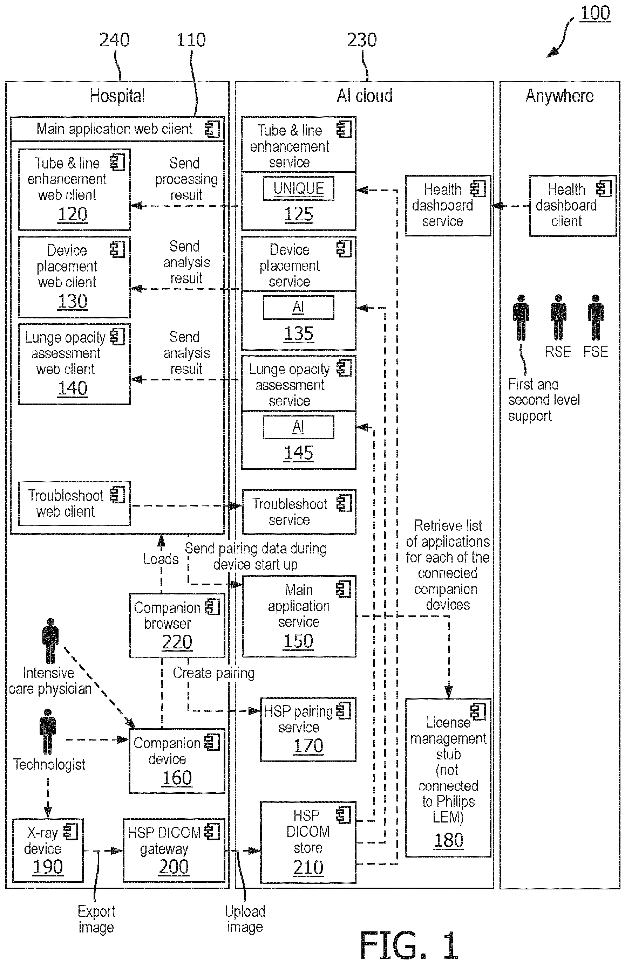

FIG. 1 is a schematic illustration of a context suite 100 according to one embodiment of the invention. The context suite may be built on top of three main components, namely an application framework, a mechanism for dynamically loading and unloading applications, and an extension of an imaging device.

The application framework typically allows for the consolidation of the output of multiple clinical algorithms, including, in many instances, AI algorithms, which can then contribute to holistic answers to clinical questions from a user. This allows the context suite concept to have a specific role in a specific clinical workflow or context. Accordingly, the application framework may include instances of a main application web client 110, which pairs with internal web clients, such as a tube and line enhancement web client 120, a device placement web client 130, and a lung opacity assessment web client 140. Sample implementations of these web clients are discussed in more detail below.

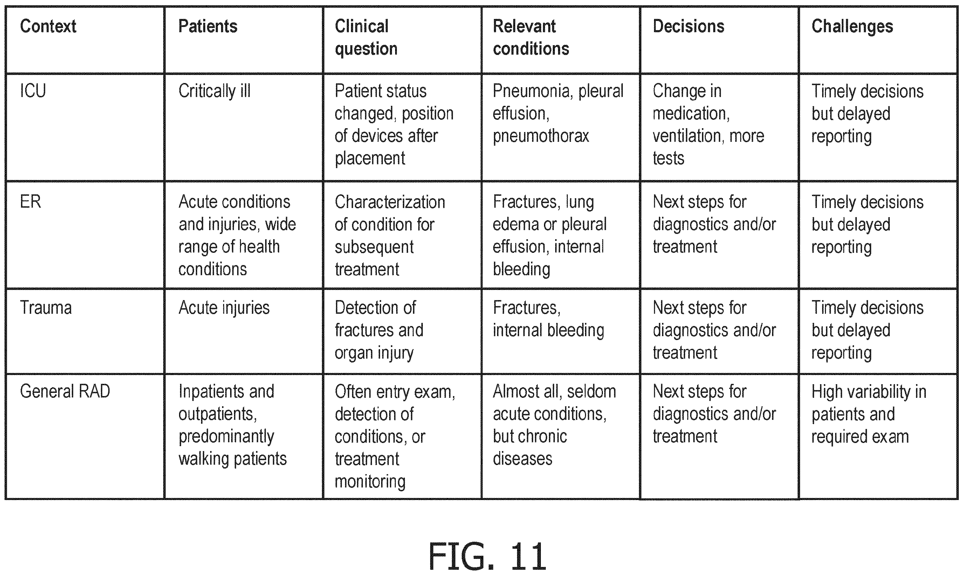

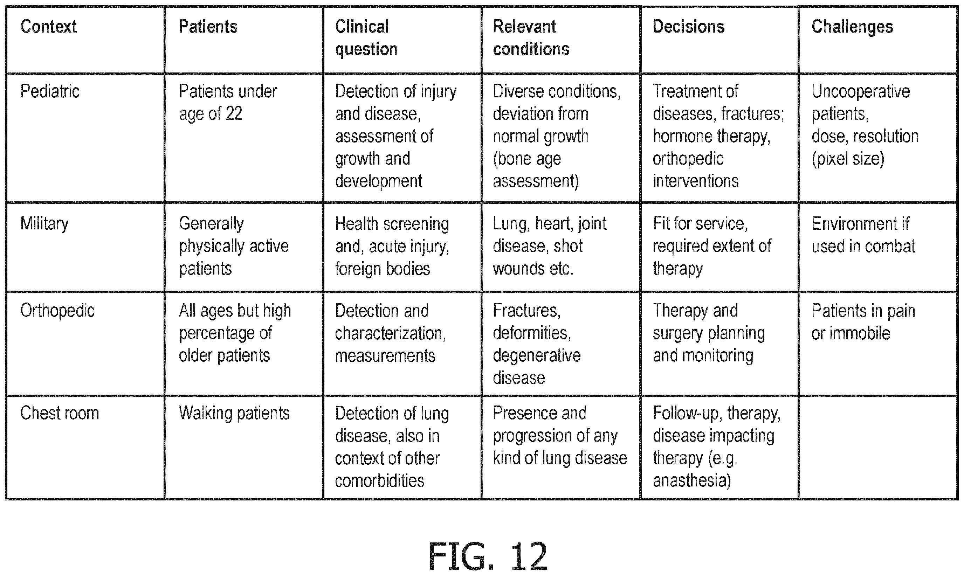

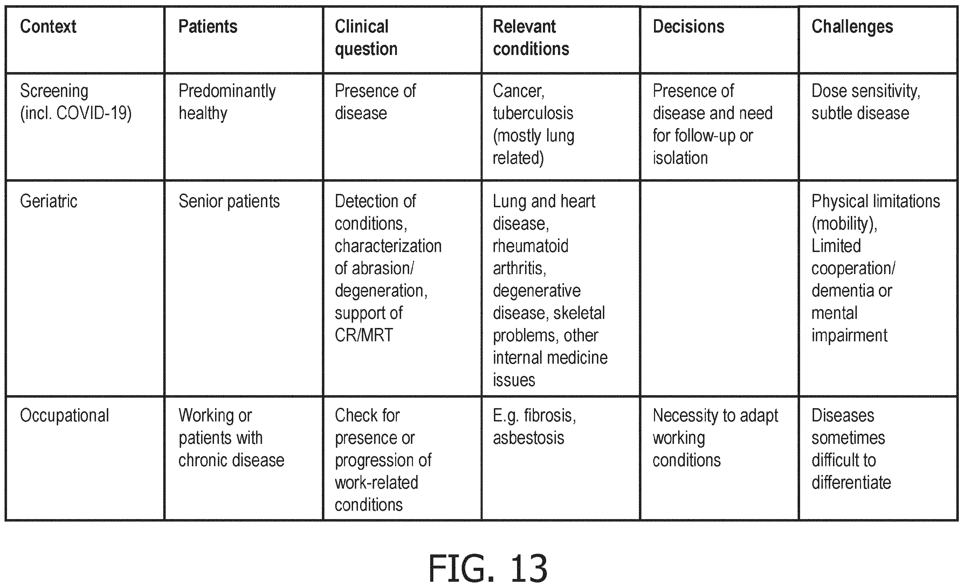

Each context specific web client 120, 130, 140 may then be supported by a corresponding processing service 125, 135, 145. The distinct web clients 120, 130, 140, may then work alongside each other or in concert to assist physicians in specific clinical scenarios. In the application framework shown, for example, the loaded web clients are provided to support ICU workflows. Other scenarios are contemplated as well, as presented in the tables of FIGS. 11-13.

The mechanism for dynamically loading and unloading applications may take the form of a main application service 150. Such a service may be utilized to initialize various components of the web client 110 and may be used to load and unload applications 120, 130, 140 into the client based on the individual needs of a user. The main application service may also pair the web client 110 with a specific user companion device 160. For example, when determined that the instance of the web client 110 is on a companion device 160 located in an ICU, the specific applications shown may be loaded and the instance of the web client 110 may be paired with the companion device by way of a pairing service 170.

In the context of a dynamic application loading framework, loading and unloading of applications by the main application service 150 may be managed by a license management service 180. Such a service may then use subscriptions that allow users to tailor the context suite to the context in which a corresponding imaging device is used, the needs of the users, and the workflow and processes implemented by the department.

Accordingly, the specific applications loaded may be guided by the context of the companion device 160 and limited by licensed software applications, or modules.

Finally, an imaging device 190 may be integrated into the context suite for use with the instance of the web client 110. Accordingly, the imaging device 190, which may be an X-ray device for retrieving conventional X-ray (CXR) images, or a computed tomography (CT) or magnetic resonance imaging (MRI) device for retrieving three-dimensional images, may be extended by computational resources outside of the console. Accordingly, the imaging device 190 may export images to an DICOM gateway 200 for uploading the images to an DICOM store 210, typically taking the form of a database or memory. The DICOM store 210 may then provide images to corresponding processing services 125, 135, 145 for supporting the web clients described implemented 120, 130, 140.

Accordingly, the context suite does not typically require any modifications to the imaging system 190, such as the X-ray system itself, instead simply changing its configuration to output images to a DICOM gateway 200. The pairing service 170 may then pair the imaging system 190 with the instance of the web client 110 such that the imaging system can be configured with the DICOM store 210 as an export target and so that the processing services 125, 135, 145 of the context suite 100 can retrieve the images upon export. In this way, the context suite 100 can provide clinical staff results of various clinical algorithms in a single tool, quickly accessible at the point of care, to support them in decision-making. It is noted that a DICOM store is described, but various other methods may be used to make images available to a context suite 100 instance. For example, in the context of a cloud implementation, such as that shown, images could be made available via S3 or direct file upload.

Various features of the context suite 100 are then leveraged in operation. In a typical implementation, the context suite web client 110 can be accessed through a companion software package 220. The companion 220 then gets installed and paired with the imaging device 190 for which it would show analysis results.

During startup, the companion device 160 leverages the companion software 220 to determine a list of applications 120, 130, 140 that should be loaded. For each application, the main application web client 110 may then create a new tab and load the appropriate web client. If the list changes after startup, the accessible software applications 120, 130, 140 may change accordingly.

In use, the system 100 described typically includes a memory for storing a plurality of instructions and, in some cases, images. The system further includes the imaging device 190 and a server 230, which may be a cloud-based server. The server 230 may receive an image from the imaging device 190 and may apply at least one image processing routine 125, 135, 145.

The system typically further includes a local device, such as the companion device 160, for displaying a processed image 510 received from the server 230. A processor is then provided that couples with the memory and is configured to execute instructions stored on the memory and implement the methods described herein.

It is noted that a system 100 is shown and described for off-premises (i.e. cloud based) or on-premises (i.e., server based) computing, which are parallel implementations. Also contemplated are on-modality implementations. Accordingly, instead of using cloud computing resources, a context suite 100 could also run on an on-premises server in the hospital or even on the imaging modality 190 itself. However, such an implementation would still typically have a connection to the cloud based server to pull corresponding applications 120, 130, 140 and their updates from the cloud or from a central server or to check which apps the customer has been subscribed for.

A DICOM gateway is typically not used if the clinical algorithms are run on the modality itself. Accordingly, in such a scenario, the image upload to the DICOM store 210 by way of the DICOM gateway 200 would not be implemented.

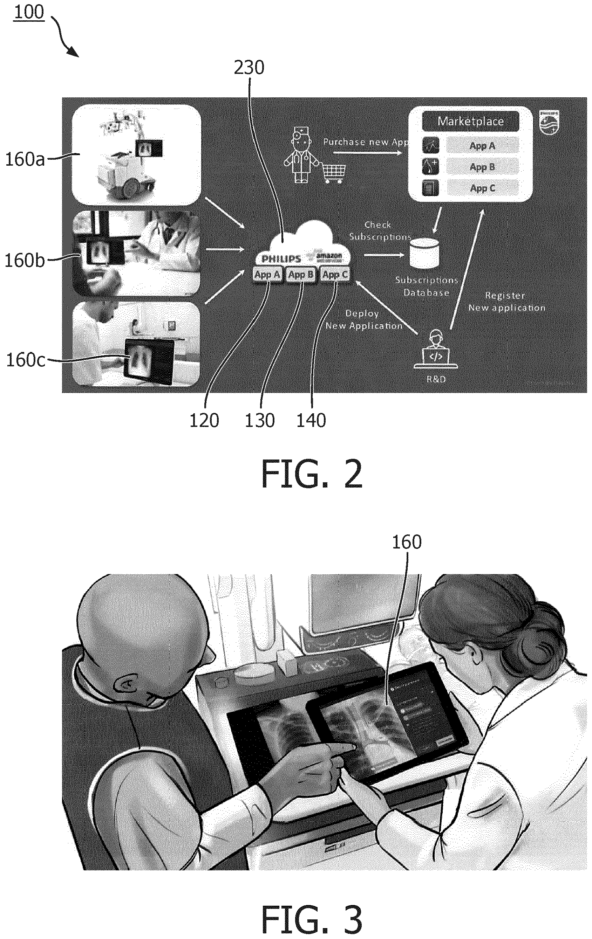

FIG. 2 provides an illustration of the provision of applications to users in the context of the context suite 100 of FIG. 1. FIG. 3 illustrates the use of the context suite 100 of FIG. 1 in context. As shown, the web application 110 can run on different companion devices 160a, b, c, depending on where it is needed. Accordingly, the companion device may be a hardware display attached to the imaging device 160a, a physicians tablet 160b, or an office computer 160c. In some embodiments, the web client 110 may operate directly in a display associated with the imaging device 190, and may not require a separate display.

During startup, the context suite 100 connects to servers 230 associated with the system, such as a cloud-based system. The relevant instance 110 of the context suite 100 may then download the application 120, 130, 140. A selection of applications to be downloaded may depend on the subscriptions the customer has purchased, or it may depend on a context for a particular companion device 160. At any time, the customer can add or cancel subscriptions. Once configured the context suite 100 may be set to automatically add or remove applications 120, 130, 140 without further human interaction. The applications 120, 130, 140 of the context suite 100 may be independently released, and thereby may be provided by third parties.

The decoupled lifecycle and an independent server 230 based backend, such as a cloud-based implementation, allows the release of additional context relevant applications after a context suite 100 has been installed in a hospital 240. It will make use of the automatic deployment that is triggered immediately after the customer has purchased a subscription for the new application.

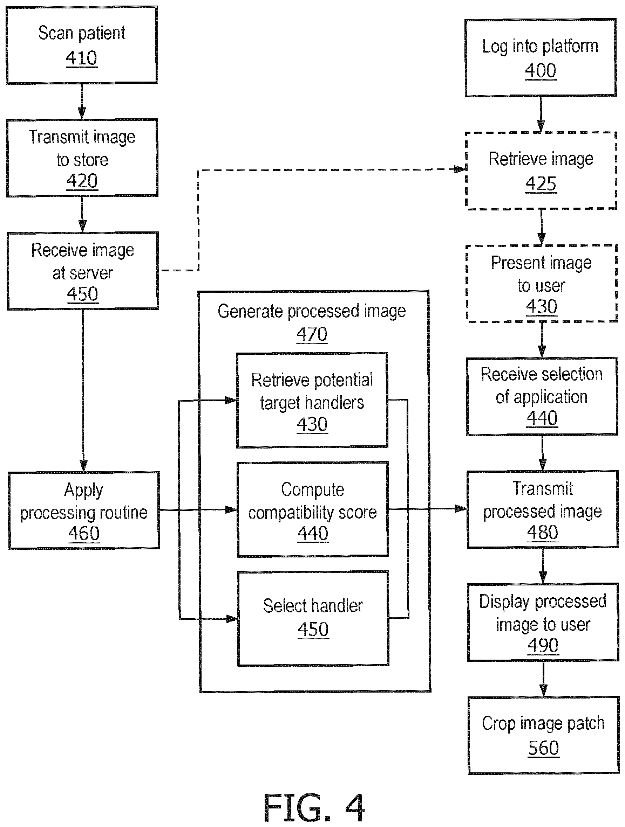

FIG. 4 provides a flow chart showing a method for contextually processing images in accordance with this disclosure. As shown, the method initially retrieves some input (400) from a user at a companion device 160 by way of the main application web client 110.

The input (at 400) may be a user logging in to the platform, for example. In parallel with such a login, a patient may be scanned (410) at an imaging device 190. As discussed above, such an imaging device 190 may be paired with the instance of the context suite 100 or it may be remotely provided. Whether the imaging device 190 is paired directly with the instance of the context suite 100 may depend on the context for a particular implementation. For example, in ICU or ER contexts, the imaging device 190 may be paired with the context suite 100 instance, while more general radiology contexts may draw on imaging from a variety of imaging devices.

Accordingly, in ICU or ER contexts, or in other scenarios where the context suite 100 is paired with an imaging device, the source of the image received at the server 230 depends on the specific local device 160 at which the contextual display application 120, 130, 140 is selected.
The scanned image is than transmitted (420) to an image store, such as the DICOM store 210 discussed above. The main application web client 110 and the underlying applications 120, 130, 140 may then draw on images stored in the DICOM store 210.

In some embodiments, once a user logs into the system (at 400) and the patient is scanned (at 410), the user is presented (430) with an initial image 500 of the user. Such an initial image 500 may be a conventional x-ray image (CXR) retrieved directly from the imaging device 100 without any initial enhancements, and may be provided for context. Alternatively, the initial image 500 may be an image with some standard processing applied. In any event, where the initial image 500 is presented to the user (430), such an image is first retrieved (425) from the DICOM store 210.

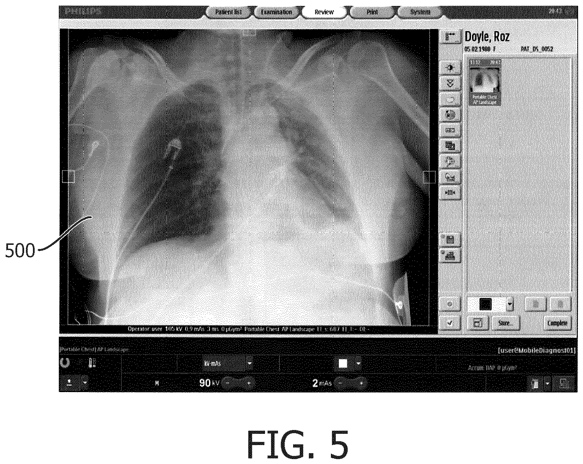

FIG. 5 shows the receipt of an image 500 from an imaging device 190 in the context of the context suite 100 of FIG. 1.

Returning to the method of FIG. 4, the user may then select any of a plurality of potential contextual display applications 120, 130, 140, and the method may receive (440) such a selection at the local companion device 160. As discussed above, the display applications 120, 130, 140 typically call for the display of a processed image at the local device 160.

Following, or in parallel, with such a selection (at 440), the server, which may be implemented as a cloud platform 230, receives (450) the image 500 from the imaging device 190, typically by way of the DICOM store 210. The method then proceeds to process (460) the image 500 at the server 230 by applying an image processing routine at a corresponding service 125, 135, 145 to generate (470) a processed image 510.

The processing routine applied (at 460) is typically based on the selected one of the plurality of potential contextual display applications 120, 130, 140 (selected at 440). Each potential contextual display application 120, 130, 140 has an associated distinct processing routine 125, 135, 145.

Once the image 500 is processed, the processed image 510 is transmitted (480) to the local device 160 for display. The processed image 510 is then displayed (490) at the local device 160 in the context of the selected contextual display application 120, 130, 140, within the main application web client 110.

In some embodiments, receipt of the images from the imaging device 190 may be sequenced differently. Accordingly, in some embodiments, a user may first log into the system (at 400) and then initiate a scan of the patient (at 410), review the initial image 500 (presented at 430), select an appropriate contextual display application 120, 130, 140 (at 440) and thereby trigger the processing of the image 500 by the corresponding service 125, 135, 145 (at 460). The resulting processed image 510 may then be transmitted (at 480) and displayed (at 490). As shown, the image processing routines may then be installed at the server 230 and implemented at the local device 160 without any required installation at the local device.

Alternatively, the scanning of the patient (at 410) may take place independently of the user's actions. Accordingly, the patient may be scanned (at 410) and the image 500 generated may be transmitted (at 420) to the DICOM store 210. Any or all of the installed services 125, 135, 145 may then proceed to process the image 500 (at 460) and generate (at 470) corresponding processed images 510. When the user then accesses the context suite 100 at the local device 160, their selection (at 440) may indicate which of the processed images 510 should be retrieved.

Further, the selection of potential contextual display applications (at 440) can take place at different times during the use of the context suite 100. During a licensing process, a hospital administrator may select which of a variety of potential contextual display applications should be licensed. Separately, during a setup process for a particular companion device 160, a user may select which of a variety of potential contextual display applications should be made available at the particular device. Such a selection may be limited to licensed contextual display applications, or a selection of an unlicensed application may be made with a request to modify license terms.

Finally, during use by a physician, the main application web client 110 may present a user with options, which may take the form of tabs. The user may then select (at 440) which tab would provide contextually valuable information based on a current medical context. Accordingly, the plurality of potential contextual display applications 120, 130, 140 operate within the context suite 100. Potential contextual display applications may then be installed interchangeably within the context suite by modifying the setup, e.g., by the department head, or by modifying the licensing arrangement, e.g., by the hospital administrator.

This approach allows the context suite 100 to be updated regularly, either as contexts change or as new products and imaging techniques are made available. Accordingly, in some embodiments, once initialized, the plurality of potential contextual display applications 120, 130, 140 are available remotely, such as at the companion device 160 and can be selected for incorporation into the context suite 100. Once selected for incorporation, the incorporation of an application allows for use of the contextual display application 120, 130, 140 within a previously installed instance of the context suite 100.

In this manner, as noted above, image processing routines, including newly created and updated image processing routines, can be installed at the server 230 and implemented at the local device 160 without any required installation at the local device. For example, an updated image processing routine may be provided for identifying a presence of an infectious disease.

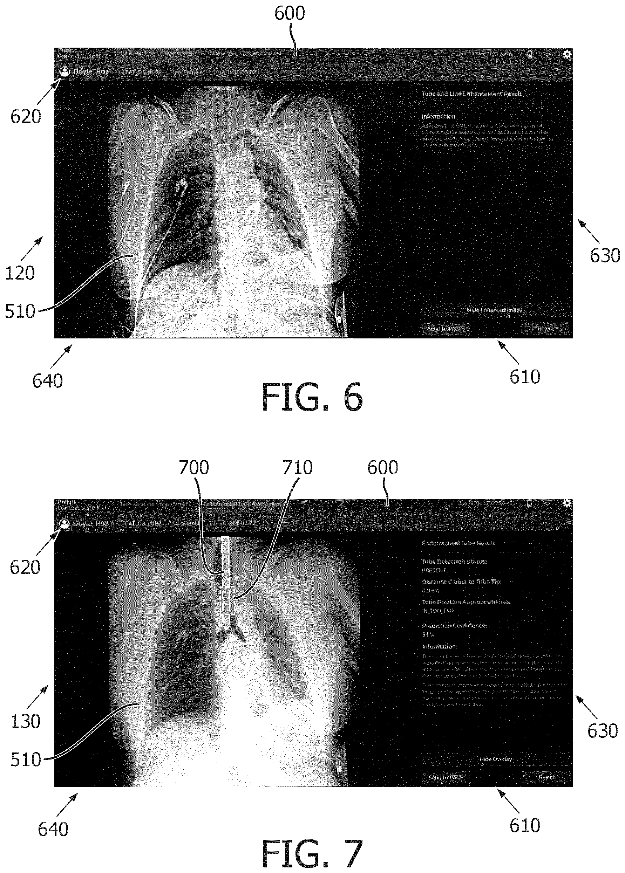

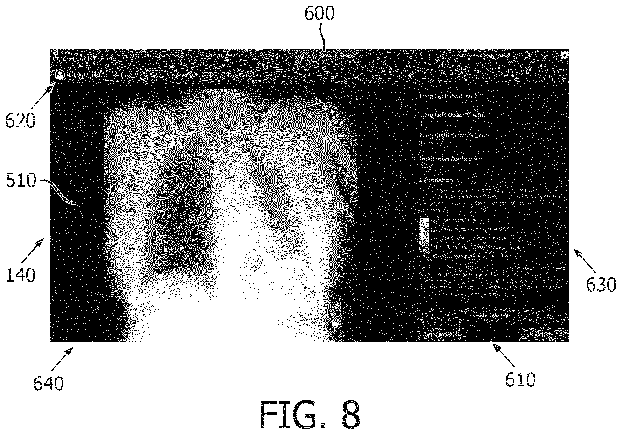

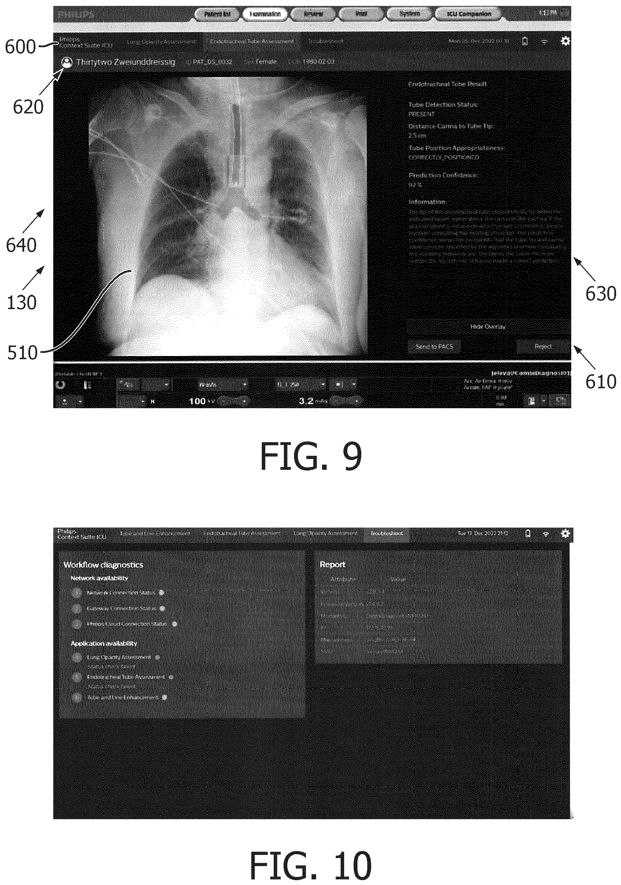

FIG. 6 shows the application of a contextually selected processing routine 125 in the context of the context suite of FIG. 1. FIG. 7 shows the application of a second contextually selected processing routine 135 in the context of the context suite of FIG. 1. FIG. 8 shows the application of a third contextually selected processing routine 145 in the context of the context suite of FIG. 1. FIG. 9 shows the application of the second contextually selected processing routine 135 in the context of a standard user interface accessible by physicians.

When potential contextual display applications are made available, a first of the potential contextual display applications 120 may be a tube and line enhancement module. In such a scenario, the display application 120 may be selected and the corresponding processing routine 125 may be applied (at 460) to generate (at 470) the processed image 510. The tube and line enhancement service 125 may then apply processing that adjusts contrast in such a way that structures of the size of catheters, tubes, and cannulas are shown with more clarity.

A second of the potential contextual display applications 130 may then be a device placement assessment module. In such a scenario, the display application 130 may be selected and the corresponding processing routine 135 may be applied (at 460) to generate (at 470) the processed image 510. The device placement assessment module 130 may assess, e.g., endotracheal tube placement. Such a processing routine 135 may detect the presence of a tube 700 in the corresponding image 500 and evaluate the position of the tube. In some embodiments, the processing routine 135 may then include segmenting the identified tube 700, identifying a target region 710 for the tube, and evaluating a position of the tube.

A third of the potential contextual display applications 140 may be a lung opacity assessment module. In such a scenario, the display application 140 may be selected and the corresponding processing routine 145 may be applied (at 460) to generate (at 470) the processed image 510. The processing routine 145 may assign a lung opacity score that describes the severity of the opacification depending on the extent of involvement by consolidation or ground-glass opacities. An overlay may then be generated in the processed image 510 to illustrate prediction confidence for different areas in the lungs.

In any event, different image processing routines 125, 135, 145 applied to generate the corresponding processed images 510 differ based on the selected contextual display application 120, 130, 140 selected. Each distinct processing routine 125, 135, 145 may be a different algorithm for processing the image 500. In some embodiments, some or all of the processing routines 125, 135, 145 may be machine learning or artificial intelligence (AI) driven processing routines. In such embodiments, the processing routines may be trained on images associated with a context related to the corresponding potential contextual display application 120, 130, 140.

Typically, the local device 160 is associated with one of a plurality of different contexts, and the contextual display applications 120, 130, 140 available vary based on such context. The exampled discussed above is based on an intensive care unit (ICU) implementation, and in such a scenario, the applications may be selected from the tube and line enhancement module 120, device placement assessment 130, and a lung opacity assessment 140 shown in FIGS. 6-9.

However, alternative groupings of applications may be designed and provided for, e.g., emergency room (ER), a trauma center, a general radiation center, a pediatric center, a military hospital, an orthopedic center, a chest room, a screening center, a geriatric center, or an occupational therapy center. In all scenarios, a plurality of potential contextual display applications are selected for incorporation into the context suite 100 based on the specific context with which the local device is associated. These alternative scenarios are shown schematically in FIGS. 11-13.

As shown in the sample displays of FIGS. 6-9, the context suite 100 typically includes two main sections, an upper area 600 generally related to the main application web client 110 having tabs for the individual applications 120, 130, 140 and setting icons, and a lower area 610 that contains the individual applications themselves, when selected.

The clinical applications 120, 130, 140 embedded in the lower area 610 of the context suite 100 may then include three main areas, namely patient information 620, viewport and status 630, and application results and actions 640. The application results and actions section 640 typically includes the display of the processed image 510.

The context suite 100 may further be embedded into a standard or proprietary medical portal, as shown in FIG. 9. The portal may be associated with a standard imaging device 190 user interface, and as such, this may support an early review of the raw image output 500, as shown in FIG. 5.

FIG. 11 shows a troubleshooting application incorporated into the context suite of FIG. 1. In some embodiments, a troubleshooting application is provided and may be accessed by a user within the context suite 100 upon receiving a selection of the same at the local device 160.

A potential challenge of server based or cloud services is that an outage can affect many customers at the same time. Therefore, self-service may be an important tool in such a platform as a service solution (PaaS) that integrates software as a service (SaaS). The troubleshooting tab will enable an operator to find out whom to contact in the event of a problem.

These three main challenges should get addressed or facilitated by a self-service / troubleshooting tab:
Provide self-service tooling and remote support for hospital staff (BioMed Engineer and IT Administrator) for minor fixes like broken cables or blocked network routes.

Faster response time, preferably pro-actively fixing issues to minimize downtime.

Economically optimized communication with customers, including indication in the Context Suite in case of known cloud issues, early detection of duplicate calls during escalation through the Philips service levels, and efficient call routing to the responsible Philips team.

In the Troubleshooting tab shown in FIG. 10, the hospital staff can see the various connection states of the components and services. To give an example: if the local network or gateway is the problem, the local IT department needs to be contacted. If clinical services from the cloud-based server are not available, as indicated here by the red lights in FIG. 11, the operator may want to contact their service provider directly. The context suite 100 solution may advise the operator through automatically generated messages in the troubleshooting tab whom to contact to resolve a problem. If there is a general issue affecting multiple customers, a general message or alert may be issued to inform the impacted customers that the IT group responsible is already aware of the problem and will provide a solution within a given time. In this way, an excessive accumulation of service calls can be avoided, and the service team can focus on solving the problem.

FIGs. 11-13 illustrate potential contexts to which the context suite of FIG. 1 can be applied.

The methods according to the present invention may be implemented on a computer as a computer implemented method, or in dedicated hardware, or in a combination of both. Executable code for a method according to the present invention may be stored on a computer program product. Examples of computer program products include memory devices, optical storage devices, integrated circuits, servers, online software, etc. Preferably, the computer program product may include non-transitory program code stored on a computer readable medium for performing a method according to the present disclosure when said program product is executed on a computer. In an embodiment, the computer program may include computer program code adapted to perform all the steps of a method according to the present disclosure when the computer program is run on a computer. The computer program may be embodied on a computer readable medium.

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the disclosure.

All examples and conditional language recited herein are intended for pedagogical purposes to aid the reader in understanding the principles of the disclosure and the concepts contributed by the inventor to furthering the art, and are to be construed as being without limitation to such specifically recited examples and conditions. Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

## Claims

1. A method for contextually processing images, comprising:
receiving a selection of one of a plurality of potential contextual display applications to display a processed image at a local device;
receiving an image from an imaging device at a server;
processing the image at the server by applying an image processing routine based on the selected one of the plurality of potential contextual display applications, wherein each potential contextual display application has an associated distinct processing routine;
transmitting the processed image to the local device for display; and
displaying the processed image at the local device in the context of the selected contextual display application.

2. The method of claim 1, wherein the receiving of the image at the server is from a DICOM store, wherein the DICOM store receives the image from the imaging device, the imaging device being a networked imaging device.

3. The method of claim 1, wherein the plurality of potential contextual display applications operate within a context suite, and wherein the potential contextual display applications are installed interchangeably within the context suite.

4. The method of claim 3, wherein upon installation of the context suite at the local device, the instance of the context suite is paired with the imaging device, such that the source of the image received at the server depends on the specific local device at which the contextual display application is selected.

5. The method of claim 3, wherein the plurality of potential contextual display applications are installed locally within the context suite, and one of the potential contextual display applications is selected based on a current medical context.

6. The method of claim 5, wherein a first potential contextual display application of the plurality is a tube and line enhancement module, and a second potential contextual display application of the plurality is a device placement assessment module, and wherein the modules are accessible interchangeably within the context suite.

7. The method of claim 3, wherein the plurality of potential contextual display applications are available remotely and can be selected for incorporation into the context suite, and wherein the incorporation of a contextual display application allows for use of the contextual display application within a previously installed instance of the context suite.

8. The method of claim 1, wherein a plurality of potential contextual display applications are selected for incorporation into the context suite based on the specific context with which the local device is associated.

9. The method of claim 1, wherein an updated image processing routine can be installed at the server and implemented at the local device without any required installation at the local device.

10. The method of claim 9, wherein the updated image processing routine is for identifying a presence of an infectious disease.

11. The method of claim 1, wherein the imaging device is a CT imaging device, an X-ray device, or an MRI device.

12. The method of claim 1, wherein each distinct processing routine is an AI processing routine trained on images associated with a context related to the corresponding potential contextual display application.

13. A system for contextually processing images, the system comprising:
a memory for storing a plurality of instructions;
an imaging device;
a server for receiving an image from the imaging device and applying an image processing routine;
a local device for displaying a processed image received from the server; and
at least one processor that couples with the memory and is configured to execute the instructions to:
receive a selection of one of a plurality of potential contextual display applications for the display of a processed image at the local device;
receive the image from the imaging device at the server;
process the image at the server by applying an image processing routine based on the selected one of the plurality of potential contextual display applications, wherein each potential contextual display application has an associated distinct processing routine;
transmit the processed image to the local device for display; and
display the processed image at the local device in the context of the selected contextual display application.

14. The system of claim 13, wherein the at least one processor is further configured to implement a troubleshooting interface upon receiving a selection of the same at the local device.

15. A non-transitory computer-readable medium for storing executable instructions, which cause a method to be performed to contextually process images, the method comprising:
receiving a selection of one of a plurality of potential contextual display applications to display a processed image at a local device;
receiving an image from an imaging device at a server;
processing the image at the server by applying an image processing routine based on the selected one of the plurality of potential contextual display applications, wherein each potential contextual display application has an associated distinct processing routine;
transmitting the processed image to the local device for display; and
displaying the processed image at the local device in the context of the selected contextual display application.
